# EUROPÄISCHE PATENTSCHRIFT

(11) **EP 0 423 679 B1**
(45) Veröffentlichungstag und Bekanntmachung des Hinweises auf die Patenterteilung: **27.04.1994**
(21) Anmeldenummer: 90119738.4
(22) Anmeldetag: 16.10.1990
(51) Int. Cl.: C07D 223/22

(54) **Verfahren zur Herstellung von 5-(Carbamoyl)-5H-dibenz[b,f]azepin**
Process for the preparation of 5-(carbamoyl)-5H-dibenz[b,f]azepines
Procédé pour la préparation de la 5-(carbamoyl)-5H-dibenz[b,f]azepine

(30) Priorität: 16.10.1989 HU 532289
(43) Veröffentlichungstag der Anmeldung: 24.04.1991
(73) Patentinhaber: ALKALOIDA VEGYESZETI GYAR, H-4440 Tiszavasvári (HU)
(72) Erfinder: Cziaky, Zoltan, H-4440 Tiszavasvari (HU); Szabo, Zoltan, H-4029 Debrecen (HU); Frank, Laszlo, Dr., H-4440 Tiszavasvari (HU); Timar, Tibor, Dr., H-4440 Tiszavasvari (HU); Galamb, Vilmos, Dr., H-4440 Tiszavasvari (HU); Bartha, Ferenc, H-4440 Tiszavasvari (HU); Korik, Piroska, H-4450 Tiszalök (HU); Horvath, Katalin, H-4440 Tiszavasvari (HU)
(74) Vertreter: Beszédes, Stephan G., Dr.

(56) Entgegenhaltungen:
- EP-A- 0 173 208
- US-A- 2 948 718

## Beschreibung

Die Erfindung betrifft ein besseres Verfahren zur Herstellung von 5-(Carbamoyl)-5H-dibenz[b;f]azepin der Formel 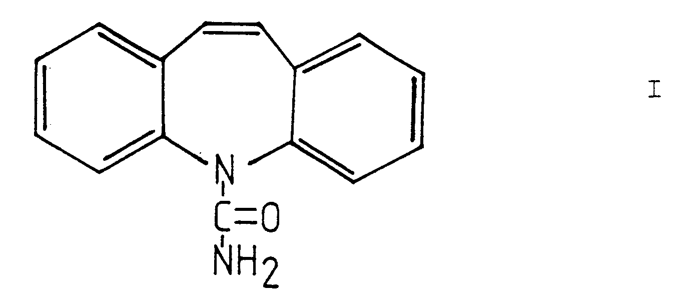
allgemein bekannt unter dem Namen Carbamazepin.

5-(Carbamoyl)-5H-dibenz[b;f]azepin kann direkt aus 5H-Dibenz[b;f]azepin (allgemein bekannter Name, Iminostilben) [dazu sind mehrere Verfahren bekannt] oder aus 10,11-Di-(hydro)-5H-dibenz[b;f]azepin (allgemein bekannter Name: Iminodibenzyl) in der Weise hergestellt werden, daß die Iminogruppe zunächst mit Phosgen chlorcarbonyliert und dann das erhaltene 5-(Chlorcarbonyl)-5H-dibenz[b;f]azepin nach Bromieren und Dehydrobromieren ammonolysiert wird. Das Bromieren kann mit elementarem Brom (zum Beispiel polnische Patentschrift 64 802, DE-OS 2 238 904, DDR-Patentschrift 133 052) oder mit 1,3-Di-(brom)-5,5-di-(methyl)-hydantoin (britische Patentschrift 1 245 506) erfolgen.

Das Bromieren mit elementarem Brom ist jedoch in technischem Maßstab beziehungsweise Industriemaßstab außerordentlich gefährlich. Ein besonderes Problem stellt die Behandlung dem freiwerdenden großen Menge Bromwasserstoff dar. Andererseits erhöht das Ersetzen des elementaren Bromes durch 1,3-Di-(brom)-5,5-di-(methyl)-hydantoin den Herstellungsaufwand bedeutend und außerdem zerfällt diese Substanz leicht. Bei beiden Verfahren erfolgt das Chlorcarbonylieren des 5H-Dibenz[b;f]azepines beziehungsweise 10,11-Di-(hydro)-5H-dibenz[b;f]azepines mit Phosgen, auf die Gefährlichkeit dieses Vorganges wird noch später näher eingegangen. Die Herstellung des 5-(Carbamoyl)-5H-dibenz[b;f]azepines durch die oben beschriebenen Verfahren ist auch außerordentlich geräteaufwendig.

Die für die Herstellung des 5-(Carbamoyl)-5H-dibenz[b;f]azepines direkt aus 5H-Dibenz[b;f]azepin bekannten Verfahren können nach den angewandten Verfahrensweisen in mehrere Gruppen eingeteilt werden.

Zur einen Gruppe gehören die Verfahren, bei denen das 5H-Dibenz[b;f]azepin mit Phosgen oder mit einer Lösung von Phosgen in Toluol chlorcarbonyliert und dann das erhaltene 5-(Chlorcarbonyl)-5H-dibenz[b;f]azepin ammonolysiert wird (US-PS 2 948 718, polnische Patentschrift 89 708, DDR-Patentschrift 126 329). Der größte Nachteil dieser Verfahren besteht darin, daß das Chlorcarbonylieren mit Phosgen erfolgt.

Aus der EP-A-0 173 208 ist ein Verfahren zur Herstellung von Tetrahydrochinolin-1-yl-carbonylchloriden bekannt, die durch Umsetzung von substituiertem Tetrahydrochinolin mit Phosgen oder Chlorameisensäuretrichlormethylester erhalten werden. Auch bei diesem Verfahren besteht der Nachteil darin, daß das Chlorcarbonylieren mit Phosgen erfolgt.

Phosgen ist - wie es allgemein bekannt ist - eine sehr giftige Substanz mit niedrigem Siedepunkt (8°C). Zur Durchführung der Umsetzung ist ein großer (5- bis 7-facher) Phosgen-Überschuß erforderlich, dessen Handhabung (besonders unter technischen beziehungsweise industriellen Bedingungen) schwierig ist und in sicherheitstechnischer Hinsicht eine besondere Verfahrenstechnik beziehungsweise Technologie erfordert. Ein weiterer Nacheil dieser bekannten Verfahren besteht darin, daß das Isolieren und Reinigen des bei der Chlorcarbonylierungs-Reaktion entstehenden 5-(Chlorcarbonyl)-5H-dibenz[b;f]azepines mit einem hohen präparativen Verlust einhergeht, und außerdem das Trennen des Reaktionsgemisches wegen des noch vorhandenen Phosgenes im Hinblick auf den Umwelt- und Unfallschutz gefährlich ist.

Die oben erwähnten Patentschriften unterscheiden sich in der Art und Weise der Durchführung der Ammonolyse voneinander. In der US-PS 2 948 718 wird die äthanolische Lösung des 5-(Chlorcarbonyl)-5H-dibenz[b;f]azepines unter Einleiten von Ammoniakgas unter Rückfluß erhitzt. Eigenen Erfahrungen zufolge ist die Verwendung von polaren Lösungsmitteln bei der Ammonolyse nicht vorteilhaft, da diese Lösungsmittel selbst bei niedriger Temperatur das Produkt verhältnismäßig gut lösen, und damit die Ausbeute sich verringert. Gemäß der DDR-Patentschrift 126 329 wird die Ammonolyse in zwei Phasen (einer wäßrigen und einer mit Wasser sich nicht vermischenden) durchgeführt. Eigenen Versuchen zufolge erhöht eine solche Durchführung der Ammonolyse die Reaktionszeit stark und ist auch hinsichtlich der Ausbeute und Qualität des Produktes nicht vorteilhaft. Im technischen beziehungsweise industriellen Maßstab verursacht ein entsprechendes Trennen der beiden Phasen ein besonderes Problem. Gemäß der polnischen Patentschrift 89 708 wird die Ammonolyse ebenfalls in zwei Phasen,und zwar unter Druck und Verwendung einer Ammoniaklösung vorgenommen. Außer den erwähnten Nachteilen besteht ein weiteres Problem darin, daß die Umsetzung in einer druckbeständigen Vorrichtung durchgeführt werden muß.

Zu einer zweiten Gruppe gehören die Verfahren, bei welchen die Bildung der Aminocarbonylgruppe ohne Phosgenisieren des 5H-Dibenz[b;f]azepines erfolgt. Gemäß einem Verfahren wird das Iminostilben mit Acylisocyanaten umgesetzt und dann wird das 5-(Carbamoyl)-5H-dibenz[b;f]azepin durch basische Hydrolyse des erhaltenen N-5-(Acyl-carbamoyl)-5H-dibenz[b;f]azepines erhalten (DE-OS 2 307 174). Der Nachteil dieses Verfahrens besteht darin, daß die Reaktion des 5H-Dibenz[b;f]azepines und der Acylisocyanate ein reversibler Vorgang ist und nur in einem bestimmten Temperaturbereich vonstatten geht. Wenn die Temperatur zu hoch ist (die Reaktion ist dann stark exotherm), treten Zerfallsprozesse in den Vordergrund. Dasselbe gilt auch für die Hydrolyse. Ein anderer bedeutender Nachteil dieses Verfahrens besteht darin, daß die technische beziehungsweise industrielle Herstellung der zur Reaktion benötigten Acylisocyanate (zum Beispiel durch Umsetzen von Acylchloriden und Silbercyanat) mit hohem Aufwand verbunden ist. Gemäß einem anderen Verfahren wird 5-(Trichloracetyl)-5H-dibenz[b;f]azepin mit Ammoniak umgesetzt (Japanische Kokai 7 733 686). Der Nachteil dieses Verfahrens besteht darin, daß das Aufbereiten und Reinigen des 5-(Trichloracetyl)-5H-dibenz[b;f]azepines mit Verlust einhergeht und ferner seine darauffolgende Ammonolyse zu 5-(Carbamoyl)-5H-dibenz[b;f]azepin gemäß den Beispielen 4 Tage in Anspruch nimmt.

Gemäß einer dritten Verfahrensart wird 5-(Carbamoyl)-5H-dibenz[b;f]azepin durch basische Hydrolyse von 5-[S-(Methyl)-isothiocarbamoyl]-5H-dibenz[b;f]azepin hergestellt (belgische Patentschrift 845 363). Die Herstellung des 5-(S-(Methyl)-isothiocarbamoyl]-5H-dibenz[b;f]azepines ist in der belgischen Patentschrift 845 362 beschrieben.

Auf Grund der obigen Patentschriften kann das 5H-Dibenz[b;f]azepin in 6 Schritten in 5-(Carbamoyl)-5H-dibenz[b;f]azepin überführt werden. Dieses Verfahren geht mit hohem präparativem Verlust einher und ist bei technischer beziehungsweise industrieller Durchführung außerordentlich geräteaufwendig und auch in sonstiger Hinsicht mit hohem Aufwand verbunden.

Gemäß einer vierten Verfahrensart wird das 5H-Dibenz[b;f]azepin mit Chlorcyan umgesetzt und das 5-(Carbamoyl)-5H-dibenz[b;f]azepin durch Behandlung des erhaltenen 5-(Cyano)-5H-dibenz[b;f]azepines mit Wasserstoffperoxyd erhalten (europäische Patentschrift 029 409). Bei der technischen beziehungsweise industriellen Durchführung dieses Verfahrens treten folgende Probleme auf: Das Chlorcyan ist eine stark giftige Flüssigkeit mit niedrigem Siedepunkt (bei Raumtemperatur gasförmig). Da die Umsetzung bei 70°C durchgeführt wird und das Chlorcyan im Überschuß verwendet wird, muß das Austreten des Chlorcyanes in die Umwelt verhindert werden, was eine besondere Vorrichtung erfordert (Ausfrieren bei -20°C). Ein weiterer Nachteil besteht darin, daß sowohl die Reaktion des 5H-Dibenz[b;f]azepines mit Chlorcyan als auch die Reaktion des 5-(Cyano)-5H-dibenz[b;f]azepines mit Wasserstoffperoxyd lange Reaktionszeiten in Anspruch nehmen (1 Tag beziehungsweise 15 Stunden).

Zusammenfassend ist zu den obigen bekannten Verfahren festzustellen, daß das 5-(Carbamoyl)-5H-dibenz[b;f]azepin in mehreren Schritten, durch Aufbereiten des Zwischenproduktes und unter Verwendung von Fall zu Fall stark giftigen, mit hohem Aufwand verbundenen Substanzen oder Substanzen mit niedrigem Siedepunkt hergestellt wird.

Der Erfindung liegt die Aufgabe zugrunde, unter Behebung der Nachteile der bekannten Verfahren ein einfacheres technisch beziehungsweise industriell leicht und sicher durchführbares, umweltfreundliches Verfahren, bei welchem von einfacher zu handhabenden und besser zugänglichen Ausgangssubstanzen ausgegangen werden kann, zu schaffen.

Das Obige wurde überraschenderweise durch die Erfindung erreicht.

Im Gegensatz zur Herstellung des 5-(Chlorcarbonyl)-5H-dibenz[b;f]azepines in den bekannten Verfahren unter Verwendung von Phosgen oder einer Lösung von Phosgen in Toluol in großem Überschuß wurde nämlich in eigenen Versuchen überraschenderweise festgestellt, daß, wenn zur Herstellung des 5-(Chlorcarbonyl)-5H-dibenz[b;f]azepines der heutzutage bereits technisch beziehungsweise industriell leicht und wirtschaftlich herstellbare, das heißt Diphosgen, verwendet wird, die Reaktion auch schon bei einem geringen, wie 20- bis 70%-igen Molüberschuß vollständig abläuft. Eine weitere überraschende Feststellung besteht darin, daß dabei das 5-(Chlorcarbonyl)-5H-dibenz[b;f]azepin so rein entsteht, daß sein Isolieren und Reinigen überflüssig ist und direkt durch Ammonolyse 5-(Carbamoyl)-5H-dibenz[b;f]azepin mit hohem Reinheitsgrad hergestellt werden kann. Bei den bekannten Verfahren wird dieses Zwischenprodukt isoliert, was eigenen Erfahrungen zufolge mit hohem Verlust einhergeht. Besonders vorteilhaft ist die Verwendung des Chlorameisensäuretrichlormethylesters wegen seines hohen Siedepunktes (bei 10 mm Hg 20°C) und seiner leichten Handhabung.

Gegenstand der Erfindung ist daher ein Verfahren zur Herstellung von 5-(Carbamoyl)-5H-dibenz[b;f]azepin der Formel 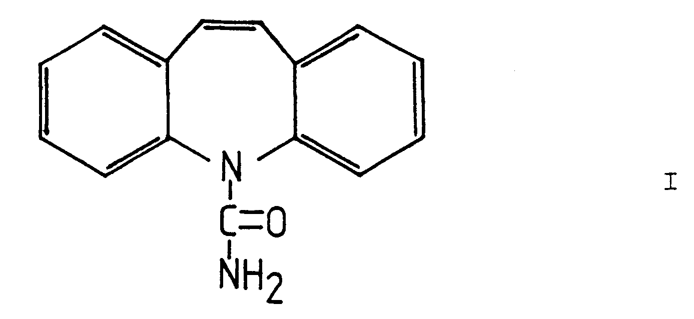
durch Chlorcarbonylieren von 5H-Dibenz[b;f]azepin der Formel 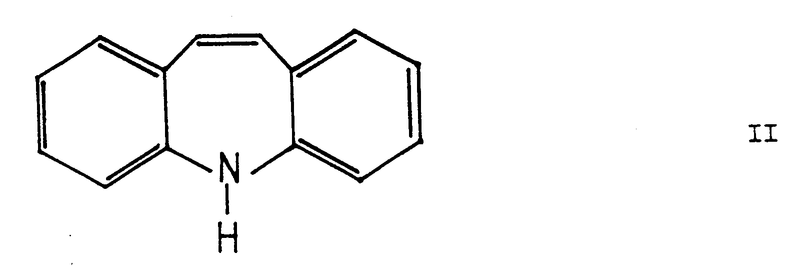
und Ammonolyse des erhaltenen 5-(Chlorcarbonyl)-5H-dibenz[b;f]azepines der Formel 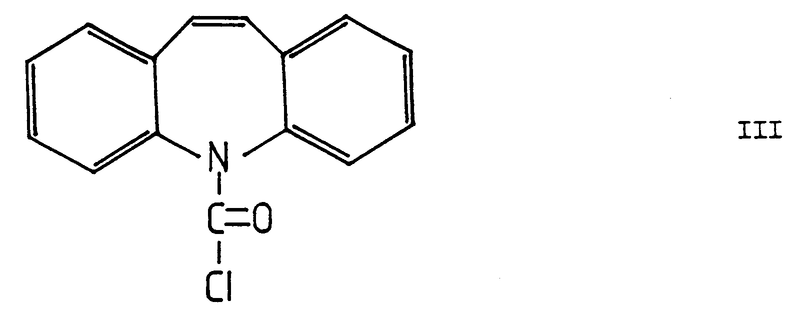
mit Ammoniakgas, welches dadurch gekennzeichnet ist, daß das Chlorcarbonylieren des 5H-Dibenz[b;f]azepines der Formel II mit Chlorameisensäuretrichlormethylester in einem apolaren aromatischen Lösungsmittel und die Ammonolyse in derselben Vorrichtung ohne Abtrennen und Reinigen des 5-(Chlorcarbonyl)-5H-dibenz[b;f]azepines der Formel III im unveränderten Reaktionsmedium (ohne Abbau des Systemes) durchgeführt wird.

Hinsichtlich des Umweltschutzes und der Wirtschaftlichkeit besteht ein besonderer Vorteil darin, daß aus dem Reaktionsgemisch nur eine geringe Menge Phosgen austritt, die leicht von einer Lauge absorbiert werden kann. Durch das erfindungsgemäße Verfahren kann einerseits der bei den bekannten Verfahren beim Isolieren des 5-(Chlorcarbonyl)-5H-dibenz[b;f]azepines auftretende Verlust vermieden werden, andererseits ist ein Trennen beziehungsweise Spalten des auch noch Phosgen enthaltenden Reaktionsgemisches nicht notwendig, was sowohl im Hinblick auf den Umweltschutz als auch unter sicherheitstechnischen Gesichtspunkten ein besonderer Vorteil ist. Beim erfindungsgemäßen Verfahren können die Chlorcarbonylierungsreaktion und die Ammonolyse in einer einzigen Vorrichtung vorgenommen werden, was gegenüber den bekannten Verfahren die technische beziehungsweise industrielle Durchführung des Verfahrens vereinfacht, seinen Gerätebedarf verringert.

Von großem Vorteil ist bei der Durchführung die Verwendung von apolaren aromatischen Lösungsmitteln, vorzugsweise Toluol oder Benzol, da sich bei der erfindungsgemäß angewandten Temperatur die gesamte Menge des 5-(Chlorcarbonyl-5H-dibenz[b;f]azepines in Lösung befindet, die gesamte Menge des bei der Reaktion entstehenden 5-(Carbamoyl)-5H-dibenz[b;f]azepines hingegen sich bei Raumtemperatur aus der Lösung absondert. Ein weiterer Vorteil besteht darin, daß das apolare aromatische Lösungsmittel, wie Toluol oder Benzol, die bei der Reaktion entstehenden Verunreinigungen in Lösung hält. Das Lösungsmittel kann mittels allgemein gebräuchlicher Verfahrensweisen rückgewonnen werden. Mit dem Ammoniakgas kann (im Falle einer entsprechenden Dispergierung) eine kurze (1- bis 3-stündige) Reaktionszeit erreicht werden, und das austretende Ammoniakgas kann bei der Reaktion erneut verwendet werden.

Zusammenfassend ist festzustellen, daß erfindungsgemäß 5-(Carbamoyl)-5H-dibenz[b;f]azepin, ausgehend von 5H-Dibenz[b;f]azepin, unter Verwendung von Chlorameisensäuretrichlormethylester und Ammoniakgas ohne Isolieren und Reinigen des 5-(Chlorcarbonyl)-5H-dibenz[b;f]azepines bei Ablaufen der Reaktion als Eintopfreaktion im Vergleich zu den bekannten Verfahren wesentlich einfacher, sicherer und wirtschaftlicher hergestellt werden kann.

Vorzugsweise werden als Lösungsmittel aromatische Kohlenwasserstoffe, gegebenenfalls mit Halogen- und/oder Alkylsubstituenten, oder deren Gemische verwendet.

Besonders bevorzugt wird beziehungsweise werden als aromatische[r] Kohlenwasserstoff(e) Benzol, Toluol, ein Xylol, ein Xylolisomergemisch oder Chlorbenzol oder deren Gemische verwendet, wobei Toluol ganz besonders bevorzugt ist.

Vorzugsweise wird zum Chlorcarbonylieren des 5H-Dibenz[b;f]azepines der Formel II der Chlorameisensäuretrichlormethylester in einem Molüberschuß von 20 bis 70%, insbesondere 30 bis 50%, verwendet.

Es ist auch bevorzugt, das Chlorcarbonylieren des 5H-Dibenz[b;f]azepines der Formel II bei 60 bis 140°C, insbesondere 80 bis 115°C, ganz besonders 80 bis 90°C, durchzuführen.

Vorzugsweise wird die Ammonolyse des 5-(Chlorcarbonyl)-5H-dibenz[b;f]azepines der Formel III durch Einleiten von Ammoniakgas bei 50 bis 90°C, insbesondere 70 bis 80°C, vorgenommen.

Es ist auch bevorzugt, die Ammonolyse des 5-(Chlorcarbonyl)-5H-dibenz[b;f]azepines der Formel III durch Einleiten eines 3- bis 5-fachen Molüberschusses von Ammoniakgas in der Weise durchzuführen, das das aus dem Reaktionsgemisch austretende überschüssige Ammoniakgas in das System zurückgeführt wird.

Ferner ist es bevorzugt, das Isolieren des 5-(Carbamoyl)-5H-dibenz[b;f]azepines der Formel I durch Kühlen auf Raumtemperatur und sein Reinigen durch Breiigmachen mit aliphatischen Kohlenwasserstoffen oder 5 bis 8 Kohlenstoffatome aufweisenden cycloaliphatischen Kohlenwasserstoffen oder deren Gemischen und darauffolgendes Waschen mit Wasser durchzuführen.

Die Erfindung wird an Hand der folgenden Beispiele näher erläutert.

### Beispiel 1

Es wurden 86,5 g (0,5 Mol) 5H-Dibenz[b;f]azepin in 1 250 ml Toluol eingewogen und auf 90°C erhitzt. Der Lösung wurde unter Rühren beziehungsweise Schütteln während 30 bis 40 Minuten 50 ml einer Lösung von 45,5 mol (0,375 Mol) Chlorameisensäuretrichlormethylester in Toluol zugetropft. Nach deren Zugabe wurde die Lösung 30 Minuten lang unter Rückfluß zum Sieden erhitzt und dann auf 70°C abgekühlt. Danach wurden während 2 Stunden etwa 3 bis 4 Mol Ammoniakgas unter kräftigem Rühren beziehungsweise Schütteln durch die Lösung geleitet. Die Suspension wurde auf Raumtemperatur abgekühlt und der feste Stoff wurde herausfiltriert, 2-mal mit 150 ml n-Hexan und 2-mal mit 500 ml Wasser gewaschen und getrocknet.
Ausbeute: 106,1 g (90,1% der Theorie).
Schmelzpunkt: 190 bis 191°C.

### Beispiel 2

Es wurden 96,5 g (0,5 Mol) 5H-Dibenz[b;f]azepin in 1 450 ml Benzol eingewogen und unter Rückfluß zum Sieden erhitzt. Der Lösung wurde unter Rühren beziehungsweise Schütteln während 30 bis 40 Minuten 50 ml einer Lösung von 39,5 ml (0,325 Mol) Chlorameisensäuretrichlormethylester in Benzol zugetropft. Nach Zugabe dieser Lösung wurde 60 Minuten lang unter Rückfluß zum Sieden erhitzt und dann auf 65 bis 70°C abgekühlt. Dann wurden während 3 Stunden etwa 3 bis 4 Mol Ammoniakgas unter kräftigem Rühren beziehungsweise Schütteln durch die Lösung geleitet. Die Suspension wurde auf Raumtemperatur abgekühlt und der feste Stoff wurde abfiltriert, 2-mal mit 150 ml Petroläther und dann 2-mal mit 500 ml Wasser gewaschen und getrocknet.
Ausbeute: 109,5 g (93% der Theorie).
Schmelzpunkt: 188 bis 190°C.

### Beispiel 3

Es wurden 96,5 g (0,5 Mol) 5H-Dibenz[b;f]azepin in 1 000 ml Xylol-Isomerengemisch eingewogen und dann auf 120°C erhitzt. Der Lösung wurde unter Rühren beziehungsweise Schütteln während 20 bis 30 Minuten 50 ml einer Lösung von 51,6 ml (0,425 Mol) Chlorameisensäuretrichlormethylester in Xylol-Isomerengemisch zugetropft. Danach wurde die Lösung 20 Minuten lang auf dieser Temperatur gehalten und dann auf 70°C abgekühlt. Darauffolgend wurden während 3 Stunden unter kräftigem Rühren beziehungsweise Schütteln etwa 3 bis 4 Mol Ammoniakgas durch die Lösung geleitet. Die Suspension wurde auf Raumtemperatur abgekühlt und der feste Stoff wurde abfiltriert und 2-mal mit 250 ml Cyclohexan und dann 2-mal mit 500 ml Wasser gewaschen und getrocknet.
Ausbeute: 100,1 g (85% der Theorie).
Schmelzpunkt: 188 bis 190°C.

### Beispiel 4

Es wurden 96,5 g (0,5 Mol) 5H-Dibenz[b;f]azepin in 1 400 ml Chlorbenzol eingewogen und dann auf 100°C erhitzt. Der Lösung wurde bei dieser Temperatur während 30 Minuten unter Rühren beziehungsweise Schütteln 50 ml einer Lösung von 51,6 g (0,425 Mol) Chlorameisensäuretrichlormethylester in Chlorbenzol zugetropft. Nach Zugabe dieser Lösung wurde 30 Minuten lang auf 120°C erhitzt und dann auf 65 bis 70°C abgekühlt. Danach wurden 3 Stunden lang unter kräftigem Rühren beziehungsweise Schütteln etwa 3 bis 4 Mol Ammoniakgas durch die Lösung geleitet. Die Suspension wurde auf Raumtemperatur abgekühlt und der feste Stoff wurde abfiltriert, 2-mal mit 300 ml n-Heptan und dann 2-mal mit 500 ml Wasser gewaschen und getrocknet.
Ausbeute: 101,6 g (86,3% der Theorie).
Schmelzpunkt: 187 bis 189°C.

## Patentansprüche

1. Verfahren zur Herstellung von 5-(Carbamoyl)-5H-dibenz[b;f]azepin der Formel 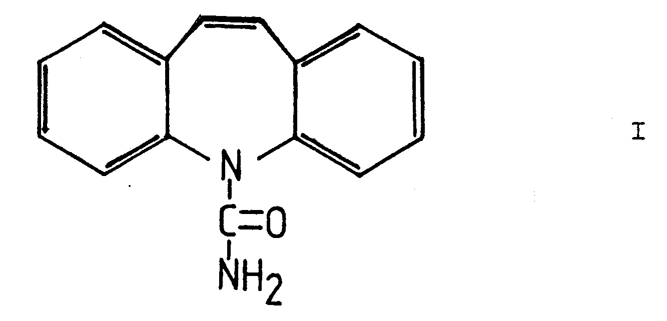 durch Chlorcarbonylieren von 5H-Dibenz[b;f]azepin der Formel 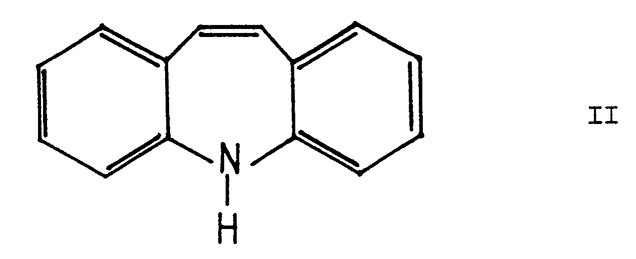 und Ammonolyse des erhaltenen 5-(Chlorcarbonyl)-5H-dibenz[b;f]azepines der Formel 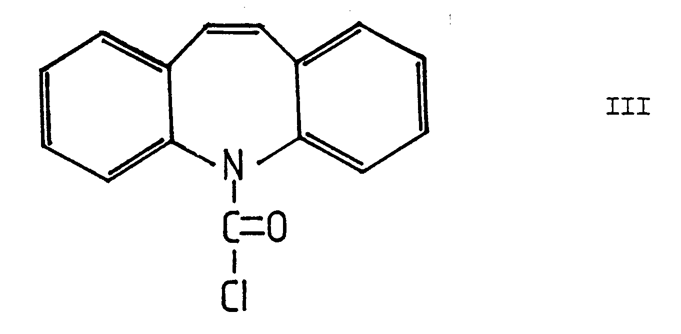 mit Ammoniakgas, dadurch gekennzeichnet, daß man das Chlorcarbonylieren des 5H-Dibenz[b;f]azepines der Formel II mit Chlorameisensäuretrichlormethylester in einem apolaren aromatischen Lösungsmittel und die Ammonolyse in derselben Vorrichtung ohne Abtrennen und Reinigen des 5-(Chlorcarbonyl)-5H-dibenz[b;f]azepines der Formel III im unveränderten Reaktionsmedium durchführt.

2. Verfahren nach Anspruch 1, dadurch gekennzeichnet, daß man als Lösungsmittel aromatische Kohlenwasserstoffe, gegebenenfalls mit Halogen- und/oder Alkylsubstituenten, oder deren Gemische verwendet.

3. Verfahren nach Anspruch 1 oder 2, dadurch gekennzeichnet, daß man als aromatische[n] Kohlenwasserstoff(e) Benzol, Toluol, ein Xylol, ein Xylolisomergemisch oder Chlorbenzol oder deren Gemische verwendet.

4. Verfahren nach Anspruch 1 bis 3, dadurch gekennzeichnet, daß man zum Chlorcarbonylieren des 5H-Dibenz[b;f]azepines der Formel II den Chlorameisensäuretrichlormethylester in einem Molüberschuß von 20 bis 70% verwendet.

5. Verfahren nach Anspruch 1 bis 4, dadurch gekennzeichnet, daß man das Chlorcarbonylieren des 5H-Dibenz[b;f]azepines der Formel II bei 60 bis 140°C durchführt.

6. Verfahren nach Anspruch 1 bis 5, dadurch gekennzeichnet, daß man das Chlorcarbonylieren des 5H-Dibenz[b;f]azepines der Formel II bei 80 bis 90°C durchführt.

7. Verfahren nach Anspruch 1 bis 6, dadurch gekennzeichnet, daß man die Ammonolyse des 5-(Chlorcarbonyl)-5H-dibenz[b;f]azepines der Formel III durch Einleiten von Ammoniakgas bei 50 bis 90°C vornimmt.

8. Verfahren nach Anspruch 1 bis 7, dadurch gekennzeichnet, daß man die Ammonolyse des 5-(Chlorcarbonyl)-5H-dibenz[b;f]azepines der Formel III durch Einleiten von Ammoniakgas bei 70 bis 80°C vornimmt.

9. Verfahren nach Anspruch 1 bis 8, dadurch gekennzeichnet, daß man die Ammonolyse des 5-(Chlorcarbonyl)-5H-dibenz[b;f]azepines der Formel III durch Einleiten eines 3- bis 5-fachen Molüberschusses von Ammoniakgas in der Weise durchführt, daß man das aus dem Reaktionsgemisch austretende überschüssige Ammoniakgas in das System zurückführt.

10. Verfahren nach Anspruch 1 bis 9, dadurch gekennzeichnet, daß man das Isolieren des 5-(Carbamoyl)-5H-dibenz[b;f]azepines der Formel I durch Kühlen auf Raumtemperatur und sein Reinigen durch Breiigmachen mit aliphatischen Kohlenwasserstoffen oder 5 bis 8 Kohlenstoffatome aufweisenden cycloaliphatischen Kohlenwasserstoffen oder deren Gemischen und darauffolgendes Waschen mit Wasser durchführt.

## Claims

1. Process for the preparation of 5-(carbamoyl)-5H-dibenz[b;f]azepine of the formula 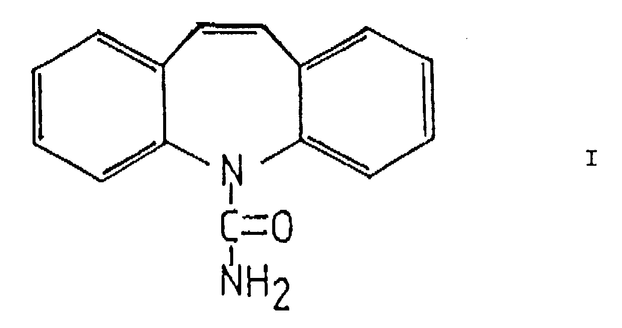 by chlorocarbonylation of 5H-dibenz[b;f]azepine of the formula 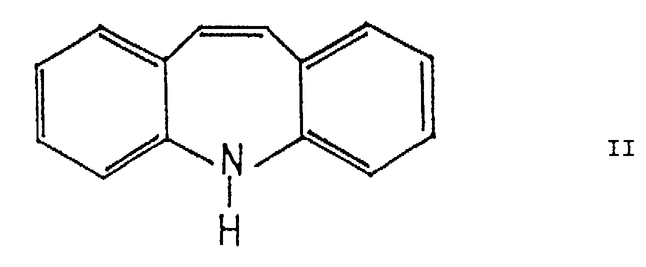 and ammonolysis of the resulting 5-(chlorocarbonyl)-5H-dibenz[b;f]azepine of the formula 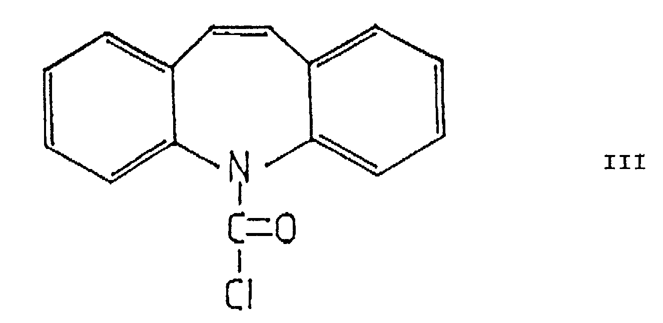 with ammonia gas, characterised in that the chlorocarbonylation of the 5H-dihenz[b;f]azepine of formula II is carried out with chloroformic acid trichloromethyl ester in a non-polar aromatic solvent and the ammonolysis is carried out in the unchanged reaction medium in the same apparatus without separating and purifying the 5-(chlorocarbonyl)-5H-dibenz[b;f]azepine of formula III.

2. Process according to claim 1, characterised in that aromatic hydrocarbons, optionally with halogen and/or alkyl substituents, or mixtures thereof, are used as solvent.

3. Process according to claim 1 or 2, characterised in that benzene, toluene, a xylene, a xylene isomor mixture or chlorobenzene or mixtures thereof is(are) used as aromatic hydrocarbon(s).

4. Process according to claims 1 to 3, characterised in that, in order to chlorocarbonylate the 5H-dibenz[b;f]azepine of formula II, the chloroformic acid trichloromethyl ester is used in a molar excess of from 20 to 70%.

5. Process according to claims 1 to 4, characterised in that the chlorocarbonylation of the 5H-dibenz[b;f]azepine of formula II is carried out at from 60 to 140°C.

6. Process according to claims 1 to 5, characterised in that the chlorocarbonylation of the 5H-dibenz[b;f]azepine of formula II is carried out at from 80 to 90°C.

7. Process according to claims 1 to 6, characterised in that the ammonolysis of the 5-(chlorocarbonyl)-5H-dibenz[b;f]azepine of formula III is carried out by introducing ammonia gas at from 50 to 90°C.

8. Process according to claims 1 to 7, characterised in that the ammonolysis of the 5-(chlorocarbonyl)-5H-dibenz[b;f]azepine of formula III is carried out by introducing ammonia gas at from 70 to 80°C.

9. Process according to claims 1 to 6, characterised in that the ammonolysis of the 5-(chlorocarbonyl)-5H-dibenz[b;f]azepine of formula III is carried out by introducing a 3- to 5-fold molar excess of ammonia gas by feeding back into the system the excess ammonia gas escaping from the reaction mixture.

10. Process according to claims 1 to 9, characterised in that the isolation of the 5-(carbamoyl)-5H-dibenz[b;f]azepine of formula I is carried out by cooling to room temperature and its purification is carried out by making it into a slurry with aliphatic hydrocarbons, or with cycloaliphatic hydrocarbons having from 5 to 8 carbon atoms, or mixtures thereof, and then washing with water.

## Revendications

1. Procédé de préparation de 5-(carbamoyl)-5H-dibenzo[b;f]azépine de formule 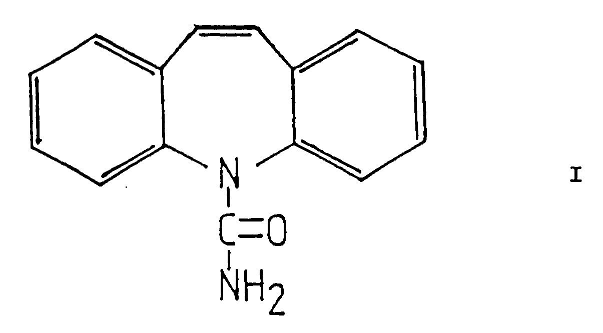 par chlorocarbonylation de 5H-dibenzo[b;f]azépine de formule 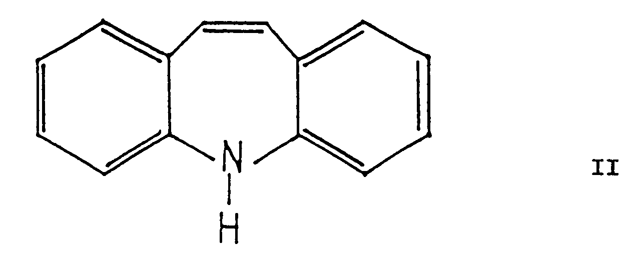 et ammoniolyse de la 5-(chlorocarbonyl)-5H-dibenzo[b;f]azépine obtenue, de formule 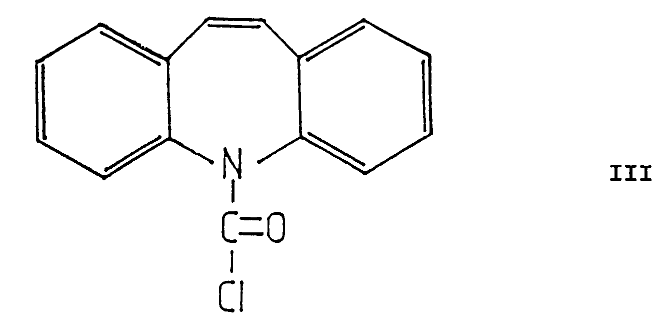 avec du gaz ammoniac, caractérisé en ce qu'on effectue la chlorocarbonylation de la 5H-dibenzo[b;f]azépine de formule II avec du chloroformiate de trichlorométhyle dans un solvant aromatique apolaire et on effectue l'ammoniolyse dans le même dispositif, sans séparation et sans purification de la 5-(chlorocarbonyl)-5H-dibenzo[b;f]azépine de formule III, dans le milieu de réaction inchangé.

2. Procédé selon la revendication 1, caractérisé en ce qu'on utilise, comme solvant, des hydrocarbures aromatiques, éventuellement avec des substituants halogénés et/ou alkylés, ou des mélanges de ceux-ci.

3. Procédé selon la revendication 1 ou 2, caractérisé en ce qu'on utilise, comme solvant(s) aromatique(s), le benzène, le toluène, un xylène, un mélange d'isomères xyléniques ou du chlorobenzène, ou des mélanges de ceux-ci.

4. Procédé selon l'une des revendications 1 à 3, caractérisé en ce qu'on utilise le chloroformiate de trichlorométhyle en un excès molaire de 20 à 70 % pour effectuer la chlorocarbonylation de la 5H-dibenzo[b;f]azépine de formule II.

5. Procédé selon l'une des revendications 1 à 4, caractérisé en ce qu'on effectue la chlorocarbonylation de la 5H-dibenzo[b;f]azépine de formule II à 60 à 140°C.

6. Procédé selon l'une des revendications 1 à 5, caractérisé en ce qu'on effectue la chlorocarbonylation de la 5H-dibenzo[b;f]azépine de formule II à 80 à 90°C.

7. Procédé selon l'une des revendications 1 à 6, caractérisé en ce qu'on effectue l'ammoniolyse de la 5-(chlorocarbonyl)-5H-dibenzo[b;f]azépine de formule III en introduisant du gaz ammoniac à une température de 50 à 90°C.

8. Procédé selon l'une des revendications 1 à 7, caractérisé en ce qu'on effectue l'ammoniolyse de la 5-(chlorocarbonyl)-5H-dibenzo[b;f]azépine de formule III en introduisant du gaz ammoniac à une température de 70 à 80°C.

9. Procédé selon l'une des revendications 1 à 8, caractérisé en ce qu'on effectue l'ammoniolyse de la 5-(chlorocarbonyl)-5H-dibenzo[b;f]azépine de formule III en introduisant du gaz ammoniac en un excès molaire valant de 3 à 5 fois, d'une manière telle qu'on recycle dans le système l'excès d'ammoniac sortant du mélange de réaction.

10. Procédé selon l'une des revendications 1 à 9, caractérisé en ce qu'on effectue l'isolement de la 5-(carbamoyl)-5H-dibenzo[b;f]azépine de formule I par refroidissement à la température ambiante, et on effectue sa purification en la transformant en une pâte avec des hydrocarbures aliphatiques ou avec des hydrocarbures cycloaliphatiques comportant de 5 à 8 atomes de carbone, ou des mélanges de ceux-ci, puis par lavage avec de l'eau.
